# EUROPEAN PATENT SPECIFICATION

(11) **EP 0 807 419 B1**
(45) Date of publication and mention of the grant of the patent: **14.08.2002**
(21) Application number: 97201442.7
(22) Date of filing: 13.05.1997
(51) Int. Cl.: A61B 17/64

(54) **Compact external fixator**
Kompakter externer Fixateur
Fixateur externe compacte

(30) Priority: 15.05.1996 IT VR960044
(43) Date of publication of application: 19.11.1997
(73) Proprietor: ORTHOFIX S.r.l., 37012 Bussolengo (VR) (IT)
(72) Inventor: Faccioli, Giovanni, 46040, Monzambano - Mantova (IT); Venturini, Daniele, 37065 - Povegliano Veronese - Verona (IT); Nelson, David, San Francisco, CA 94117 (US)
(74) Representative: Botti, Mario

(56) References cited:
- EP-A- 0 481 664
- WO-A-91/11151
- WO-A-94/06364
- WO-A-94/23662
- DE-A- 3 722 595
- DE-U- 9 319 433
- GB-A- 2 255 284
- US-A- 4 621 627

## Description

### Field of application

This invention relates to an external fixator which can be used in the field of bone surgery and in particular for the treatment of fractures in small bones, such as fractures of the first metatarsal, the wrist, the heel, and in general fractures in the bones of small children.

### State of the art

Small axial external fixators such as for example the external axial fixator model no. 30000 produced and marketed by the applicant and described and claimed in European patent no. 11,258 and in US patent 4,621,627 are known.

These known fixators generally comprise an elongatable central body with clamps attached to the longitudinal ends thereof by means of pins or bone bolts through corresponding ball joints which can be immobilised in a predetermined orientation. Each joint comprises a ball formation which is integral with a clamp or the central body and is acted upon by a locking bush pushed by an eccentric which can be rotated from the exterior by means of a suitable tool. The central body comprises two or more telescopic members which can move with respect to each other and which can be immobilised in the required position by means of suitable locking members. The form of fixator described above is produced in several sizes, one of which is used to reduce and stabilise fractures in small children and in the smallest bones of adults, such as for example the wrist or heel.

However, even this type of fixator has some recognised disadvantages, among which should be mentioned in particular the opacity of the central body to X-rays, which prevents multilateral X-rays examination of fractures during reduction and in the course of subsequent inspections.

Another disadvantage of known fixators lies in their bulkiness, due to the fact that the stop blocks and eccentric locking devices are placed between the central body and the corresponding clamps, and therefore increases the length of the fixator and the minimum distance between the bone bolts, reducing the possibilities for adjustment of the central body.

A further disadvantage lies in the relatively large weight of conventional fixators, especially when treating fractures of the wrist and in general fractures in small children and old people.

Yet another disadvantage lies in the possibility of relative torsion between the telescopic part of cylindrical shape about their longitudinal axis when these parts are not immobilised to allow limited movement of the stumps, and to accelerate new marginal growth in the fracture.

WO 9423662 discloses an external fixator whose central body comprises a male and a female member, both made of plastics material, i.e. an X-rays transparent material.

WO 94-06364 discloses an external fixator with two telescoping members with adequate X-ray transparency.

### Description of the invention

The main object of the present invention is to eliminate the aforementioned disadvantages by providing an external fixator which permits X-ray examination of the fracture from all viewpoints, including therefore through the fixator, while showing, at the same time, reliable characteristics in respect of torsional rigidity and immobilisation of the orientation of the joints.

A further object is to provide an external fixator which is particularly suitable for the treatment of fractures in the small bones of adults and in general in. the bones of small children.

Another object is to provide a fixator which has the feature of being appreciably light.

These and other objects are achieved by an external fixator according to the appended claims.

### Brief description of the figures

Further features and advantages will be more apparent in the light of the detailed description of a preferred but non-exclusive embodiment of the external fixator according to the invention which is illustrated by way of a non-restrictive example with reference to the appended table of drawings in which:

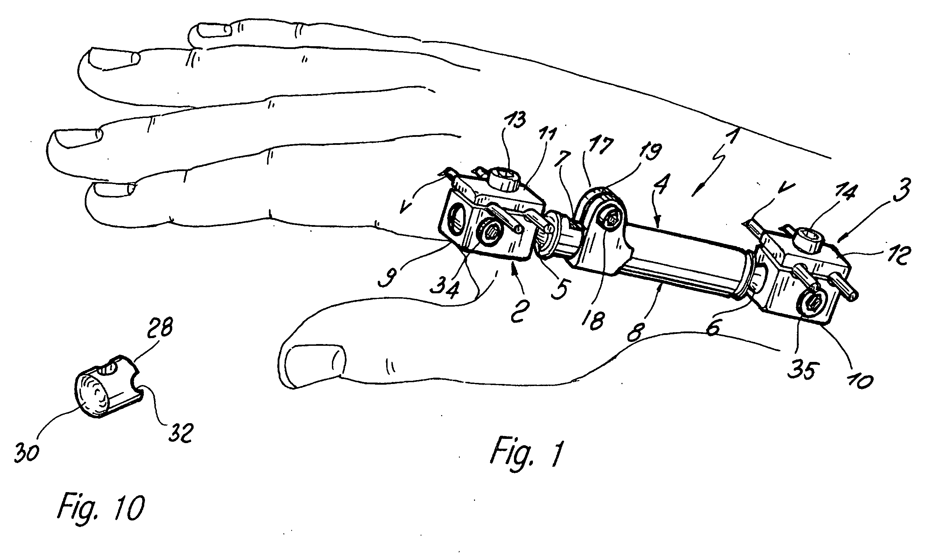

Fig.1 shows a general perspective view of an external fixator according to the invention fitted in the vicinity of an articulation of the wrist ;

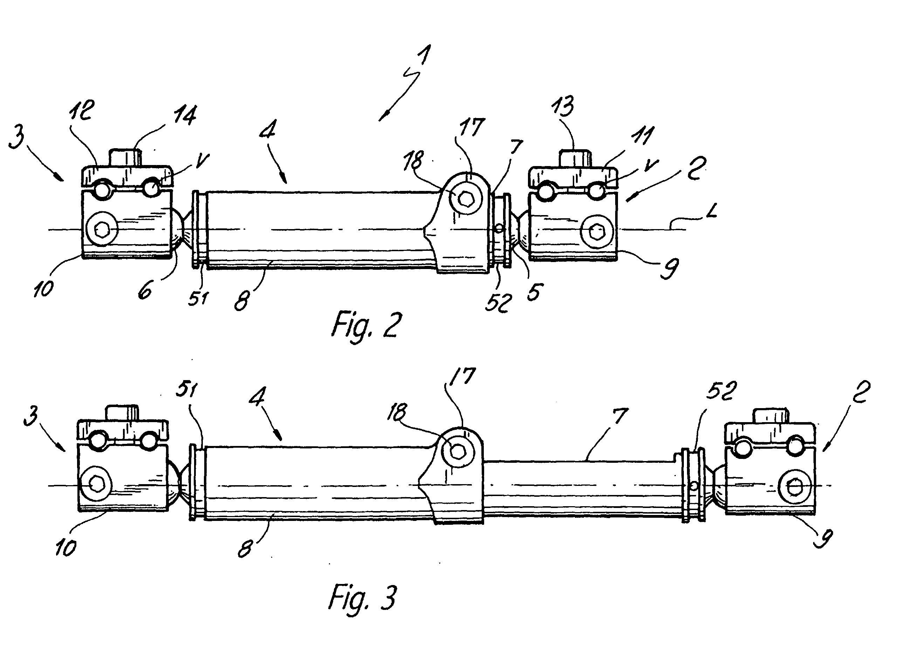

Fig.2 represents a side elevation view of the fixator in Fig. 1 in the fully retracted position;
Fig. 3 illustrates a side elevation view of the fixator in Fig. 1 in the fully extended position ;

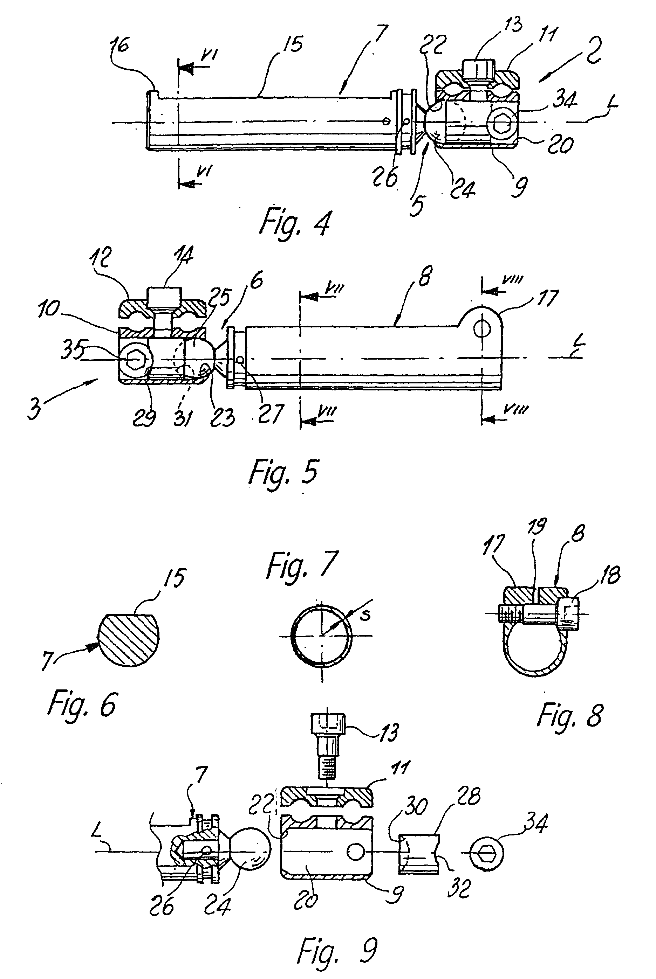

Fig. 4 shows a side view of a first detail of the above figures in partial cross-section along an axial longitudinal plane ;
Fig. 5 shows a side view of a second detail of the above figures partly in cross-section along an axial longitudinal plane;
Fig. 6 shows a view in transverse cross-section along the plane of the line VI-VI of the detail in Fig. 4 ;
Fig. 7 shows a view in transverse cross-section along the plane of line VII-VII in the detail of Fig. 5 ;
Fig. 8 shows a view in transverse cross-section along the plane of line VIII-VIII in the detail of Fig. 5 ;
Fig. 9 shows an exploded view in partial cross-section along an axial plane of a clamp in Fig. 4 ;
Fig. 10 shows a perspective view of a detail of the clamp in Fig. 9 ;
Fig. 11 shows a side view of the fixator of Fig. 1 coupled to a tensioning device ;
Fig. 12 shows a side view of the independent tensioning device of Fig. 11;
Fig. 13 is a front view of the tensioning device of Fig. 12.

### Detailed description of a preferred embodiment

With reference to the aforesaid figures, an external axial fixator according to the invention, indicated in general by reference number 1, comprises a pair of clamps 2, 3 for securing bolts or pins V which have been first fixed by the surgeon in the stumps of the fracture, for example in the wrist joint, connected to a central elongatable body 4 by means of corresponding end joints 5, 6.

Central body 4 may be formed of two members, respectively one male member 7 and one female member 8, of an approximately cylindrical shape, having a circular transverse cross-section, which are telescopically coupled in such a way that they can slide over each other along a common longitudinal axis L varying the overall length of central body 4.

Clamps 2, 3 are preferably formed of corresponding principal portions 9, 10 of approximately prismatic or parallelepiped shape connected to corresponding plates 11, 12 by means of bolts 13, 14 to secure bone pins V.

According to the invention, telescopic members 7, 8 are at least partly X-rays transparent to allow viewing of the fracture even through central body 4. One of the telescopic members, in particular male member 7, is made of a material which is highly X-rays transparent.

The highly X-rays transparent material may be a thermoplastic resin with an amorphous or semicrystalline structure selected from the polyethers, polysulphonates, polyoxymethylenes or polyetherimides.
Preferably female telescopic member 8 is constructed of a material which is substantially opaque to X-rays, such as for example an aluminium alloy, and has lateral walls of a relatively reduced thickness such as to be at least partly X-rays transparent.

From calculations and tests it has been possible to show that if a female telescopic member 8 is constructed with lateral walls of thickness S not exceeding approximately 1 mm then the central body as a whole becomes sufficiently X-rays transparent.

In order to prevent the relative rotation of telescopic members 7, 8 and therefore of bone clamps 2, 3 while the fixator is allowed to move, suitable anti-rotation means are provided.
In particular, male telescopic member 7 has a flat 15 which extends over almost its entire length, leaving an end projection 16.

At the free end opposite clamp 3, female member 8 has a lateral projection 17 in which a partly threaded hole is provided to house a transverse screw 18 of which the smooth part acts together with longitudinal flat 15 of member 7 to prevent rotation of the latter about longitudinal axis L.

Appropriately, lateral projection 17 has a central slit 19 of predetermined length so as to provide the end of female member 8 with a certain amount of radial resiliency. Bolt 18 which passes through lateral projection 17 can tighten the end of female member 8 immobilising male member 7 in the required position. In addition to this, bolt 18 comprises an end stop with respect to end projection 16, preventing involuntary separation between member 7 and member 8.

It will be noted that the main prismatic portions 9, 10 of both clamps 2, 3 have axial through cavities 20, 21 with corresponding spherical restrictions 22, 23 which interact with ball joints 24, 25 fixed to corresponding telescopic members 7, 8 by means of locking screws 26, 27.
Locking cylindrical blocks 28, 29 are housed in through cavities 20, 21 and have at one end hemispherical seats 30, 31 acting against ball joints 24, 25 and at the opposite end cylindrical diametric seats 32, 33 facing outwards.

Transverse eccentric pins 34, 35 are rotatably supported at the distal ends of principal portions 9, 10 of clamps 2, 3 so as to interact with corresponding locking blocks 28, 29 on their diametric seats 32, 33. By rotating eccentrics 34, 35 hemispherical seats 30, 31 of the blocks are forced against the opposing ball joints 24, 25 in such a way as to immobilise them in the desired orientation.

A reduction in the overall length of the fixator is achieved through this arrangement of the eccentrics on the clamps, in particular towards their outer ends, making use of the structure of the clamp itself. Thus the maximum distance between the clamps, and therefore between bone bolts V, is less than in the past, permitting optimum use of the fixator for the wrist joint, and in general for small bones and in children.

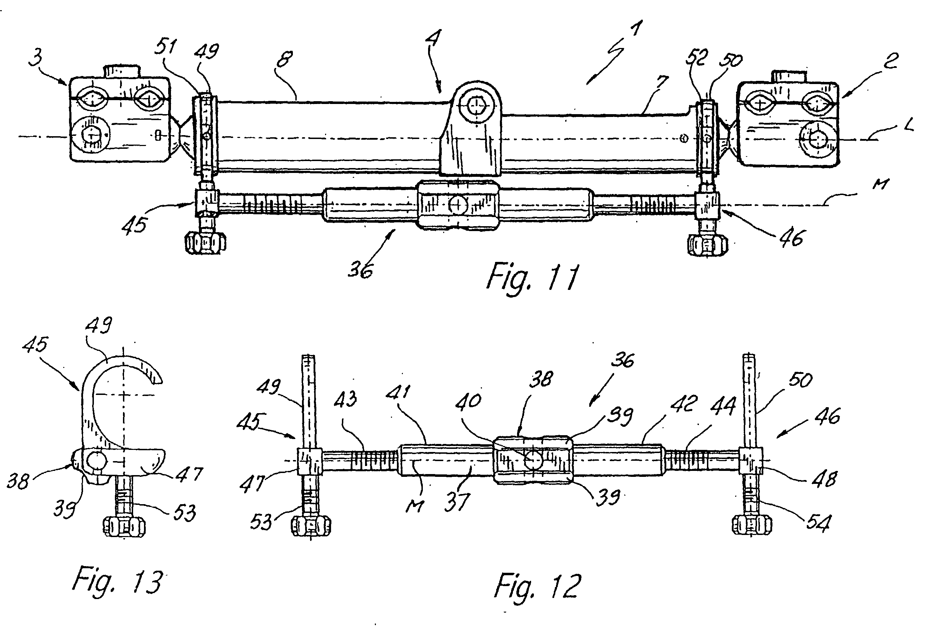

In order to allow a distraction or compression of the fracture fixator 1 is provided with means for coupling to a tensioning device which is generally indicated with the reference number 36 and is clearly illustrated in Fig. 11-13.

Tensioning device 36 comprises a sleeve 37 with a longitudinal axis M which has a substantially cylindrical expansion 38 formed on the outer surface thereof and provided with longitudinal gripping grooves 39 and with a central hole 40 for insertion of a pin-like tool to allow rotation of the sleeve 37. The end portions 41, 42 of the sleeve are internally threaded with opposite threads to receive corresponding threaded rods 43, 44 carrying at the opposite ends thereof securing means 45, 46 for rigidly coupling with fixator 1. Securing means 45, 46 are essentially formed by approximately prismatic supports 47, 48 from which hook formations 49, 50 with centres equidistant from the axis M extend. Hook formations 49, 50 can be easily inserted into cylindrical grooves 51, 52 formed at the outer ends of telescopic members 7, 8 of central body 4 in such a manner that axis M of the tensioning device lays substantially parallel to axis L of the fixator 1.

Securing means 45, 46 are rigidly coupled to fixator 1 by means of corresponding screws 53, 54 passing through transverse holes of supports 47, 48 having axis directed towards the centres of their hook formations 49, 50.

The use of the fixator will be obvious in the light of the foregoing description.

After bolts V have been fitted into the stumps of the fracture, bolts 13, 14 and eccentrics 35, 36 in the clamps, together with stop screw 18 in the central body are released. After this the length of the fixator is adjusted in such a way that bolts V are inserted between plates 11, 12 and the principal portions 9, 10 of the clamps, bolts 13 and 14 and eccentrics 34, 35 are then screwed up immobilising the orientation of the clamps and screw 18 is screwed up to fix the length of the central body.

Once the fracture has been reduced a distraction or compression of the focus can be exerted by means of tensioning device 36, specifically by inserting hook formations 49, 50 into the cylindrical grooves 51, 52 of the telescopic members 7, 8. The sleeve 37 can be rotated acting either with the fingers on the expansion 38 or with a pin-like tool on the central hole 40 to bring threaded rods 43, 44 closer or further away as much as needed.

As a result of the X-ray transparency of the central body it is possible to perform the entire operation of reducing the fracture, exposing it to X-rays even from the side on which the fixator is located, with greater ease of handling and a reduction in exposure times.

The tensioning device can be coupled to the fixator in an extremely easy manner thanks to the open structure of the hook formation of the securing means that facilitates the use of the device even in a narrow space.
The fixator according to the invention is susceptible of many modifications and variants which fall within the scope of the appended claims. Dimensions, shapes and materials may be altered without going beyond the scope of the invention.

## Claims

1. A compact external fixator which is particularly suitable for the treatment of fractures in small bones and in small children, comprising a pair of clamps (2, 3) for bone bolts (V) connected by means of immobilisable ball joints (5, 6) to a central body (4) of adjustable length comprising at least one male member (7) and one female member (8) which are attached telescopically, **characterised in that** one of the said telescopic members is constructed of a material which is highly X-rays transparent and the other of the said telescopic members is constructed of a substantially radio-opaque rigid material having lateral walls of a thickness (S) which is reduced so that they are at least partly X-rays transparent, so as to permit radioscopy of the fracture through the central body (4).

2. An external fixator according to claim 1, in which the said highly X-rays transparent material is a thermoplastic resin with an amorphous or semicrystalline structure.

3. An external fixator according to claim 2, in which the said thermoplastic resin is selected from polyethers, polysulphonates, polyoxymethylenes or polyetherimides.

4. An external fixator according to claim 1, in which the said substantially radio-opaque material is an aluminium-based metal alloy.

5. An external fixator according to any one of the preceding claims, in which said telescopic member (8) constructed of a substantially radio-opaque rigid material is the female member.

6. An external fixator according to claim 5, in which the wall of the said member (8) of radio-opaque material has a maximum thickness of approximately 1 mm.

7. An external fixator according to claim 1, in which means (7, 15, 17, 18) are provided for immobilising rotation between the male telescopic member (7) and the female member (8).

8. An external fixator according to claim 7, in which the said means immobilising rotation comprise a longitudinal flap (15) formed on the male telescopic member (7) which has a substantially circular transverse cross-section.

9. An external fixator according to claim 8, in which the said means immobilising rotation comprise a lateral projection (17) formed at the end of the female telescopic member (8) opposite the corresponding clamp and designed to house a transverse immobilising screw (18) which interacts with the longitudinal flat (15) on the male telescopic member (7) in such a way as to prevent rotation of the latter about the longitudinal axis (L) of the central body.

10. An external fixator according to claim 9, in which the said lateral projection (17) has a longitudinal central part (19) which confers radial elasticity on the end of the telescopic member (8) in which it is formed, and the said end can be resiliently closed by means of the said securing screw ( 18) to selectively immobilise the male telescopic member (7).

11. An external fixator according to claim 1, in which each clamp (2, 3) comprises a substantially prismatic principal portion (9, 10) with an axial through cavity (20, 21) in which a ball joint (24, 25) attached to a corresponding telescopic member (7, 8) is housed.

12. An external clamp according to claim 11, in which a stop block (28, 29) is inserted into a corresponding axial through cavity (20, 21), the said block (28, 29) having a first substantially hemispherical seat (30, 31) at one longitudinal end which is capable of interacting with a corresponding ball joint (24, 25) and at the opposite end a diametric cylindrical seat (32, 33).

13. An maternal fixator according to claim 12, in which a transverse eccentric pin (34, 35) is rotatably supported at the outer end of each clamp (2, 3) to interact with a corresponding block (28, 29) on the diametric seat (32, 33) of the latter to immobilise the said ball joint (24, 25).

14. An external fixator according to claim 1, in which each clamp (2, 3) comprises a plate (11, 12) which can be anchored to the said principal portion (9, 10) in order to immobilise one or more transverse bone bolts (V) with the latter.

15. An external fixator according to claim 1, in which said central body (4) has means for removable coupling to a tensioning device (36) for exerting a distraction or compression on the fracture.

16. An external fixator according to claim 15, in which said tensioning device (36) comprises a pair of securing means (45, 46) attached to the ends of corresponding counter-threaded rods (43, 44) screwed in end portions (41, 42) of a rotatable sleeve (37).

17. An external fixator according to claim 16, in which said securing means (45, 46) have hook formations (49, 50) for insertion in complementary shaped grooves (51, 52) formed at the opposite ends of said telescopic members (7, 8) and corresponding locking screws (53, 54) acting towards the centres of said hook formations (49, 50).

## Patentansprüche

1. , Kompakter externer Fixateur, der besonders zur Behandlung von Brüchen kleiner Knochen oder bei kleinen Kindern geeignet ist, umfassend ein Paar Klemmen (2, 3) für Knochenbolzen (V), die mittels sicherbarer Kugelgelenke (5, 6) mit einem zentralen Körper (4) einstellbarer Länge verbunden sind, der mindestens ein männliches Element (7) und ein weibliches Element (8), die teleskopisch angebracht sind, umfasst, **dadurch gekennzeichnet, dass** eines der teleskopischen Elemente aus einem Material hergestellt wird, das in hohem Maße für Röntgenstrahlen durchlässig ist, und das andere der teleskopischen Elemente aus einem im wesentlichen strahlungsundurchlassigen starren Material hergestellt wird und Seitenwände mit einer Dicke (S) aufweist, die so verringert ist, dass diese zumindest teilweise durchlässig für Röntgenstrahlen sind, um so eine Radioskopie des Bruchs durch den zentralen Körper (4) hindurch zuzulassen.

2. Externer Fixateur nach Anspruch 1, wobei das in hohem Maße für Röntgenstrahlen durchlässige Material ein thermoplastisches Harz mit einer amorphen oder halbkristallinen Struktur ist.

3. Externer Fixateur nach Anspruch 2, wobei das thermoplastische Harz aus Polyethern, Polysulphonaten, Polyoxymethylenen oder Polyetherimiden ausgewählt ist.

4. Externer Fixateur nach Anspruch 1, wobei das im wesentlichen strahlungsundurchlässige Material eine Metalllegierung auf Aluminiumbasis ist.

5. Externer Fixateur nach einem der vorhergehenden Ansprüche, wobei das teleskopische Element (8), das aus einem im wesentlichen strahlungsundurchlässigen starren Material hergestellt wird, das weibliche Element ist.

6. Externer Fixateur nach Anspruch 5, wobei die Wand des Elements (8) aus strahlungsundurchlässigem Material eine maximale Dicke von ungefähr 1 mm aufweist.

7. Externer Fixateur nach Anspruch 1, wobei Mittel (7, 15, 17, 18) zur Sicherung der Drehung zwischen dem männlichen teleskopischen Element (7) und dem weiblichen Element (8) zur Verfügung gestellt werden.

8. Externer Fixateur nach Anspruch 7, wobei die die Drehung sichernden Mittel eine Längseinebnung(15) umfassen, die auf dem männlichen teleskopischen Element (7) gebildet wird, das einen im wesentlichen kreisförmigen transversalen Querschnitt aufweist.

9. Externer Fixateur nach Anspruch 8, wobei das die Sicherung sichernde Mittel einen seitlichen Vorsprung (17) umfasst, der an dem Ende des weiblichen teleskopischen Elements (8) gegenüber der entsprechenden Klemme gebildet und so konstruiert ist, dass er eine querverlaufende Sicherungsschraube (18) aufnimmt, die mit der Längseinebnung(15) auf dem männlichen teleskopischen Element (7) so zusammenwirkt, dass eine Drehung des letzteren um die Längsachse (L) des zentralen Körpers verhindert wird.

10. Externer Fixateur gemäß Anspruch 9, wobei der seitliche Vorsprung (17) einen zentralen Längsteil (19) aufweist, der dem Ende des teleskopischen Elements (8), in dem es gebildet ist, radiale Elastizität verleiht und das Ende federnd mittels der Sicherungsschraube (18) geschlossen werden kann, um das männliche teleskopische Element (7) selektiv zu sichern.

11. Externer Fixateur gemäß Anspruch 1, wobei jede Klemme (2, 3) einen im wesentlichen prismatischen Hauptteil (9, 10) mit einer axialen Durchgangsöffnung (20, 21) aufweist, in der ein Kugelgelenk (24, 25), das an einem entsprechenden teleskopischen Element (7, 8) angebracht ist, untergebracht wird.

12. Externe Klemme gemäß Anspruch 11, wobei ein Sperrblock (28, 29) in eine entsprechende axiale Durchgansgöffnung (20, 21) eingefügt ist, wobei der Block (28, 29) an einem Längsende einen ersten im wesentlichen halbkugelförmigen Sitz (30, 31), der mit einem entsprechenden Kugelgelenk (24, 25) zusammenwirken kann, und am gegenüberliegenden Ende einen diametrischen zylindrischen Sitz (32, 33) aufweist.

13. Externer Fixateur gemäß Anspruch 12, wobei ein querverlaufender außermittiger Stift (34, 35) drehbar an dem äußeren Ende jeder Klemme (2, 3) so gehaltert ist, dass er mit einem entsprechenden Block (28, 29) auf dem diametrischen Sitz (32, 33) des letzteren zusammenwirkt, um das Kugelgelenk (24, 25) zu sichern.

14. Externer Fixateur gemäß Anspruch 1, wobei jede Klemme (2, 3) eine Platte (11, 12) umfasst, die mit dem Hauptteil (9, 10) verankert werden kann, um eine oder mehrere querverlaufende Knochenbolzen (V) mit letzterem zu sichern.

15. Externer Fixateur gemäß Anspruch 1, wobei der zentrale Körper (4) Mittel zum auswechselbaren Koppeln an eine Spannvorrichtung (36) aufweist, um auf den Bruch eine Distraktion oder Kompression auszuüben.

16. Externer Fixateur gemäß Anspruch 15, wobei die Spannvorrichtung (36) ein Paar Sicherungsmittel (45, 46) umfasst, die an die Enden der entsprechenden Gegen-Gewindestangen (43, 44) angefügt sind, die in Endteile (41, 42) einer drehbaren Muffe (37) eingeschraubt sind,

17. Externer Fixateur gemäß Anspruch 16, wobei die Sicherungsmittel (45, 46) Hakenbildungen (49, 50) zum Einsetzen in komplementär geformten Nuten (51, 52), die an den gegenüberliegenden Enden der teleskopischen Elemente (7, 8) angeformt sind, und entsprechende Halteschrauben (53, 54), die in Richtung auf das Zentrum der Hakenbildungen (49, 50) wirken, aufweisen.

## Revendications

1. Fixation externe compacte particulièrement appropriée pour le traitement de fractures de petits os ou chez de jeunes enfants, comprenant deux attaches par serrage (2, 3) pour boulons de chirurgie osseuse (V) reliées au moyen d'articulations à rotule immobilisables (5, 6) à un corps central (4) de longueur réglable comprenant au moins un élément mâle (7) et un élément femelle (8) fixés de manière télescopique, **caractérisée en ce qu'**un desdits éléments télescopiques est construit à partir d'un matériau hautement transparent aux rayons X et l'autre desdits éléments télescopiques est construit à partir d'un matériau rigide sensiblement opaque aux rayons X comportant des parois latérales d'une épaisseur (S) réduite de sorte qu'ils sont transparents au moins partiellement aux rayons X, de façon à permettre la radioscopie de la fracture à travers le corps central (4).

2. Fixation externe selon la revendication 1, dans laquelle ledit matériau hautement transparent aux rayons X est une résine thermoplastique dotée d'une structure amorphe ou semi-crystalline.

3. Fixation externe selon la revendication 2, dans laquelle ladite résine thermoplastique est sélectionnée parmi les polyéthers, les polysulfonates, les polyoxyméthylènes ou les polyétherimides.

4. Fixation externe selon la revendication 1, dans laquelle ledit matériau sensiblement opaque aux rayons X est un alliage métallique à base d'aluminium.

5. Fixation externe selon l'une quelconque des revendications précédentes, dans laquelle ledit élément télescopique (8) construit à partir d'un matériau rigide sensiblement opaque aux rayons X est l'élément femelle.

6. Fixation externe selon la revendication 5, dans laquelle la paroi dudit élément (8) constitué d'un matériau opaque aux rayons X a une épaisseur maximale d'approximativement 1 mm.

7. Fixation externe selon la revendication 1, dans laquelle des moyens (7, 15, 17, 18) sont prévus pour immobiliser la rotation entre l'élément télescopique mâle (7) et l'éléme femelle (8).

8. Fixation externe selon la revendication 7, dans laquelle lesdits moyens d'immobilisation anti-rotation comprennent un méplat longitudinal (15) formé sur l'élément télescopique mâle (7) qui présente une section transversale sensiblement circulaire.

9. Fixation externe selon la revendication 8, dans laquelle lesdits moyens d'immobilisation anti-rotation comprennent une saillie latérale (17) formée à l'extrémité de l'élément télescopique femelle (8) opposée à l'attache par serrage correspondante et conçue pour loger une vis d'immobilisation transversale (18) qui interagit avec le méplat longitudinal (15) sur l'élément télescopique mâle (7) de façon à empêcher toute rotation de ce dernier autour de l'axe longitudinal (L) du corps central.

10. Fixation externe selon la revendication 9, dans laquelle ladite saillie latérale (17) comporte une partie centrale longitudinale (19) conférant une élasticité radiale sur l'extrémité de l'élément télescopique (8) dans laquelle elle est façonnée, et ladite extrémité peut être fermée de façon élastique au moyen de ladite vis de fixation (18) afin d'immobiliser sélectivement l'élément télescopique mâle (7).

11. Fixation externe selon la revendication 1, dans laquelle chaque attache par serrage (2, 3) comprend une partie principale sensiblement prismatique (9, 10) dotée d'une cavité traversante axiale (20, 21) dans laquelle est logée une articulation à rotule (24, 25) fixée à un élément télescopique correspondant (7, 8).

12. Attache par serrage externe selon la revendication 11, dans laquelle un bloc d'arrêt (28, 29) est inséré dans une cavité traversante axiale correspondante (20, 21), ledit bloc (28, 29) comportant un premier siège sensiblement hémisphérique (30, 31) à une extrémité longitudinale susceptible d'interagir avec une articulation à rotule correspondante (24, 25), et un siège cylindrique diamétral (32, 33) à l'extrémité opposée.

13. Fixation externe selon la revendication 12, dans laquelle un axe excentrique transversal (34, 35) est supporté mobile en rotation à l'extrémité extérieure de chaque attache par serrage (2, 3) de façon à interagir avec un bloc correspondant (28, 29) sur le siège diamétral (32, 33) de ce dernier afin d'immobiliser ladite articulation à rotule (24, 25).

14. Fixation externe selon la revendication 1, dans laquelle chaque attache par serrage (2, 3) comprend une plaque (11, 12) pouvant être ancrée à ladite partie principale (9, 10) pour immobiliser un ou plusieurs boulons de chirurgie osseuse transversaux (V) avec cette dernière.

15. Fixation externe selon la revendication 1, dans laquelle ledit corps central (4) comporte un moyen de raccordement amovible à un dispositif de tension (36) destiné à exercer une distraction ou une compression sur la fracture.

16. Fixation externe selon la revendication 15, dans laquelle ledit dispositif de tension (36) comprend deux moyens de fixation (45, 46) fixés aux extrémités de tiges contre-filetées correspondantes (43, 44) vissées dans des parties d'extrémité (41, 42) d'une douille mobile en rotation (37).

17. Fixation externe selon la revendication 16, dans laquelle lesdits moyens de fixation (45, 46) comportent des structures en forme de crochets (49, 50) devant être insérées dans des gorges de forme complémentaire (51, 52) formées aux extrémités opposées desdits éléments télescopiques (7, 8) et des vis de verrouillage correspondantes (53, 54) agissant vers les centres desdites structures en forme de crochets (49, 50).
